# EUROPEAN PATENT APPLICATION

(11) **EP 0 882 428 A2**
(43) Date of publication of application: **09.12.1998**
(21) Application number: 98304182.3
(22) Date of filing: 27.05.1998
(51) Int. Cl.: A61B 17/12

(54) **Intravascular occlusion device**

(30) Priority: 05.06.1997 US 870149
(71) Applicant: Arterial Vascular Engineering, Inc., Santa Rosa, California 95403 (US)
(72) Inventor: Williams, Michael S., Santa Rosa, CA 95409 (US)
(74) Representative: Bayliss, Geoffrey Cyril

(57) **Abstract**

Occlusion of vessels, particularly small diameter vessels, is effected with an intravascular occlusion device comprising an anchor (10, 10', 30, 60) and an occlusive material or materials (16, 18, 44). Embodiments of the anchor include self-expanding (10, 60) or mechanically expandable (10', 30) frames comprising a plurality of wire-like members (12, 12')or a generally cylindrical, tubular-shaped member (32). Examples of the occlusive materials include filamentary strands or fiber bundles (16), and/or a non-compliant or inelastic closure member (18, 44). The strands or fiber bundles (16) are secured to the anchor by tying or adhesive. The non-compliant occlusion member (18, 44) is connected to the anchor by tying, molding, coating, adhesive, induction heating, welding or heat shrinking. The fiber bundles (16) are preferably made from material which induces a thrombogenic response. Both the fiber bundles (16) and the inelastic occlusion element (18, 44) may be coated or impregnated with thrombogenic materials or agents. Additionally, the surface of the anchor can be treated to increase the thrombogenic response of the occlusion device.

## Description

### FIELD OF THE INVENTION

The present invention relates to a method and device for permanently occluding a body lumen. And, in particular, to a device for carrying, positioning and maintaining an occlusive material at a site within the intravascular system. More specifically, within small diameter vessels such as are present in the cerebrovascular tree.

### BACKGROUND OF THE INVENTION

There are a variety of conditions where indicated treatment calls for the permanent occlusion of a vessel or lumen within the human body. Related aspects thereof, concerning endovascular therapy, include: controlling internal bleeding, limiting bleeding during surgery, relieving vessel wall pressure in proximity to an aneurysm, eliminating communication between venous and arterial vasculature (i.e., arteriovenous malformation) and occluding blood supply to tumors. Examples of non-vascular applications include therapeutic ureteric occlusion, fallopial occlusion, pulmonary occlusion and septal defects occlusion.

Several approaches and devices have been employed to produce blockage or occlusion of vessels. Such approaches include the use of thrombosis producing particles, adhesives, detachable balloons, coils or coiled wire, and a stent with a flexible closure member affixed thereto. There are drawbacks, however, associated with each of these approaches.

Thrombosis producing particles suffer from inaccurate placement. Such particles are typically released and then carried by blood flow to a point where the vessel diameter is equal to or less than the diameter of the particle. While this may be suitable for use in blocking flow to a tumor, it results in the loss of control over the point of occlusion. Also, temporary occlusion with recanalization of the vessel after occlusion can result.

Adhesives refers to liquid compounds such as isobutyl-cyanoacrylate, injected into a vessel which polymerize upon contact with blood to solidify against the vessel wall. There is very little control over the placement of the adhesive due to the cure rate of the adhesive within the bloodstream. Further, there is the risk of misdirection of the adhesive into a parent artery or other non-targeted vessel.

Disadvantages associated with balloon occlusion devices arise from their somewhat unpredictable and hazardous nature. Due to the stiffness of their delivery system, they cannot be advanced to more tortuous, distal regions, and therefore, are limited to larger vessels. Balloon occlusion devices also present the risk of premature detachment from their delivery systems. Furthermore, these balloons can deflate or, on the other hand, rupture the vessel in which it is placed.

Coils and other particulates used to occlude vessels have the disadvantage of lack of control or ability to be positioned accurately. Further, they are prone to migration once ejected out of the delivery catheter into the vessel. An additional disadvantage is that they often fail to produce complete occlusion of the target vessel.

A stent with a flexible closure member attached thereto has also been posited as an endovascular means for occluding flow through a vessel. Stents are medical implant devices designed and conventionally employed for the treatment of chronic restenosis or other intravascular narrowing. Stents are typically delivered to an affected area in a vessel and then expanded to maintain the passageway patent. Thus, stents are designed to perform a function which is antithetic to intravascular occlusion.

The flexible closure member, attached to the stent, physically blocks a vessel passageway to effect occlusion. As the flexible or elastomeric member stretches, however, it will cause an increase in the recoil of the stent. Therefore, the stent would have to be oversized to prevent migration, increasing the risk of injury or dissection of the vessel. Moreover, due to the stress imposed by the stretchable material of the stent device, the device can disengage from the vessel wall after a period of time.

Extra-vascular (surgical) treatment of aneurysms involve clipping the neck of the aneurysm, and performing suture-ligation of the neck or wrapping the aneurysm. These procedures are intrusive and risky, with high rates of mortality and morbidity.

Accordingly, there exists a need for an effective intravascular occlusion device, particularly for occluding intracranial vessels. More specifically, one which effects rapid and total occlusion in a single device, can be accurately positioned and does not migrate once positioned. And an occlusion device which is capable of being advanced into the severe tortuosity such as that characterized by the cerebrovascular tree.

### SUMMARY OF THE INVENTION

The present invention is directed to an improved intravascular occlusion device that is accurately and fixedly positioned and accomplishes permanent occlusion with a single device. Further, the invention is capable of rapid and total occlusion with little or no chronic revascularization. Additionally, the invention is capable of occluding parent and feeder vessels. And can be advanced to smaller, more tortuous vessels.

An occlusion device embodying the features of the instant invention comprises an anchor with a substantially non-compliant member and/or filamentary strands, fibers or fiber bundles attached thereto. The occlusive properties of the device are attributed to mechanical, rheological and thrombogenic means. These multiple mechanisms ensure occlusive efficacy.

The anchor can take on the configuration of any number of frames which are capable, upon delivery, of expanding into engagement with the vessel for permanent placement of the occlusion device at a predetermined site in the vessel. Particular embodiments of the frame include a plurality of wire-like members or an expandable generally cylindrical, tubular-shaped member. The various frames are preferably fabricated from a material selected form the group consisting essentially of stainless steel, nickel, titanium, tantalum, cobalt-chromium, gold, copper and alloys thereof.

The filamentary strands, fibers or fiber bundles are preferably made form materials which induce a thrombogenic response. Specifically, the filamentary strands employed in the present invention are preferably selected from the group consisting of silk, cotton, polyethylene, polyethylene terephthalate, wool, collagen yarn, cellulose and the like. The non-compliant member is preferably selected from the group of materials consisting essentially of polyethylene, polyethylene terephthalate, polyarylenesulfide and polyamides.

In accordance with the inventive method, a vessel is permanently occluded by disposing the occlusion device, comprising the anchor and non-compliant membrane and/or fibers or fiber bundles, at a predetermined location within the vessel. The inventive intravascular occlusion device can be inserted into a vessel and delivered to a predetermined location with any type of conventional delivery system.

The invention also contemplates the use of a coating for enhancing thrombogenicity or having other utility, such as drug delivery or the like. One manner in which the present invention contemplates drug delivery is by incorporation of the compound into the polymer, comprising the fibers and/or non-compliant membrane, forming a monolithic drug/polymer matrix.

### BRIEF DESCRIPTION OF DRAWINGS

For a more complete understanding of the features, aspects, and advantages of the present invention, reference is now made to the following description, appended claims, and accompanying drawings wherein:
Figures 1a through 1c are perspective views of a first embodiment of the inventive intravascular occlusion device;
Figures 2a through 2c are perspective views of a second embodiment of the inventive intravascular occlusion device;
Figures 3a through 3c represent a third embodiment of the inventive intravascular occlusion device shown in side view;
Figures 3d through 3f are a schematic representation of an exemplary delivery system and mode of use for the inventive intravascular occlusion device shown in Figures 3a through 3c; and
Figures 4a and 4b depict an alternative embodiment to the balloon-expandable device depicted in Figures 3a through 3f.

The invention and its various embodiments are best understood by now turning to the following detailed description, the drawings for which are included for purposes of illustration and not by way of limitation.

### DETAILED DESCRIPTION OF THE PREFERRED EMBODIMENTS

The inventive intravascular occlusion device provides significant advancements in the field of surgically precise therapy for intravascular occlusion. The invention provides new therapeutic endpoints beyond those currently in use for endovascular treatments employing coils, balloons, particles, cyanoacrylates and the like.

The present invention enables the accurate and reliable formations of bridges for postponement of, and improved outcome of, surgery and, in some cases, results beyond palliative treatment. The intravascular occlusion device of the present invention permits surgically precise therapy, particularly, in treating: dural fistulas; intracranial aneurysms; arteriovenous malformations (both plexiform nidus and fistulae); carotid cavernous fistulas; and various other embolization techniques and local drug delivery applications. The inventive device enjoys particular applicability in occluding intracranial vessels having small diameters.

The intravascular occlusion device of the present invention comprises an anchor and an occlusive element. The anchor can take the form of: a frame comprising a plurality of wire-like members; or a generally cylindrical, tubular-shaped member. The cylindrical, tubular-shaped anchor is similar in construction to what is typically referred to in the medical devices art as a "stent." Stents are generally employed for mechanically keeping affected vessels open after completion of an angioplasty procedure. Contrary to the known, indicated use for stents, however, the instant invention utilizes a construction similar to a stent for intravascular occlusion.

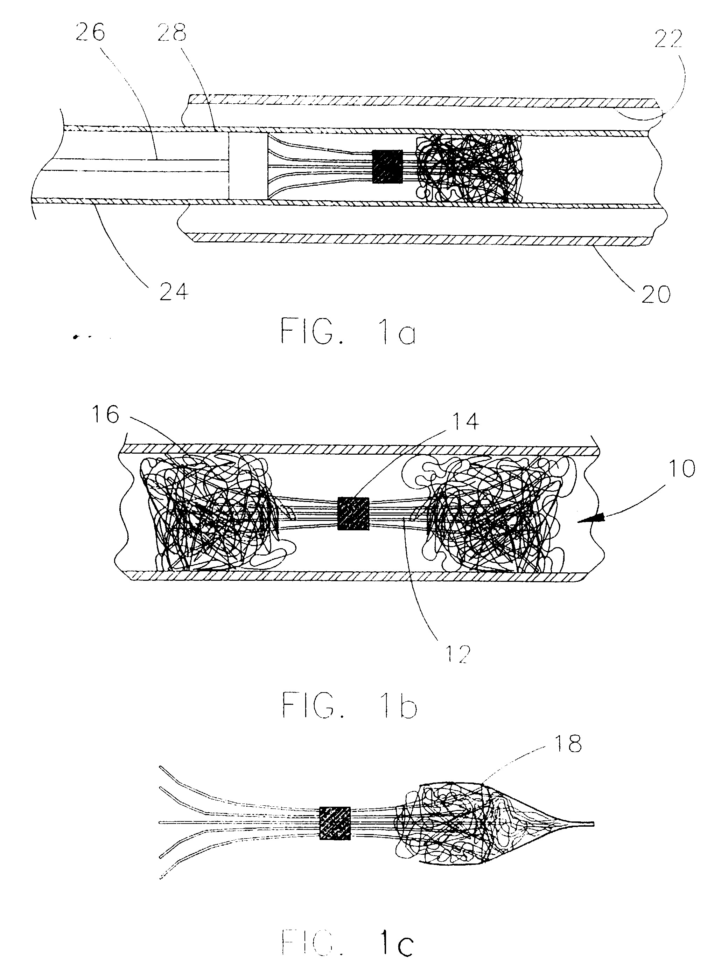

Adverting to Figures 1a-c, a first embodiment of the intraluminal device for permanently occluding fluid flow through vessel 20 is depicted. The device comprises a frame generally designated by reference numeral 10. Frame 10 includes a plurality of wire-like members 12 held together by connecting element 14. Wires 12 can be made from any suitable materials such as stainless steel, nickel, titanium, nickel-titanium, Nitinol, tantalum, cobalt-chromium, copper, gold and the like or any alloys thereof.

Wires 12 have a preferred thickness in the range of 1-10 mils. Although, thicknesses less than 1 mil and greater than 20 mils are contemplated by the present invention. Connecting element 14 is preferably a radiopaque marker band, crimped onto wires 12, for facilitating, under fluoroscopy, the identification of the position of the occlusion device. Marker band 14 can be made from any material which provides the desired radiopacity, for example, gold, platinum, tantalum, iridium, and alloys thereof.

Radiopaque marker band 14 can also welded or soldered onto wires 12. Alternatively, wires 12 may be welded or soldered together and at least a portion of the surface of frame 10 can be coated or plated with a radiopaque material.

Filamentary strands, fibers or fiber bundles 16 are intertwined about and around wires 12 or they can be woven around wires 12 prior to crimping marker band 14 about wires 12, thereby fixing the fiber bundles to the frame. The filamentary strands employed in the present invention can be any known type or configuration capable of effecting thrombogenic occlusion when disposed in a vessel. Examples of such filamentary strands include polymers such as nylon, polyester, polyethylene, polypropylene, polyurethane, polyethylene terephthalate, and the like, natural fibers such as silk, collagen yarns, cotton, wool, cellulose and the like, as well as and metals. Each element can be in the form of a monofilament or yarns, stretched or unstretched. Although specific filamentary strands are disclosed, any filamentary strands or other thrombogenic-inducing elements capable of causing thrombogenic occlusion can be employed as the occlusive element of the instant invention.

Figure 1c shows a non-compliant or inelastic membrane 18 for occluding flow. Membrane 18 is fabricated from substantially non-compliant material, which is capable of mechanically occluding vessel 20 when deployed therein. Examples of suitable materials include polymers such as polyethylene, polyethylene terephthalate, polyarylenesulfide, polyphenolsulfide, polyamides and the like. Membrane 18 may be attached to frame 10 in any suitable manner such as by thermal bonding, heat shrinking, coating, adhesives, welding, or induction heating. While Figure 1c also depicts fibers 16 intertwined around and about wires 12, it should be understood, however, that occlusion of flow can be achieved in this embodiment without fibers 16.

As an alternate to the embodiment depicted in Figure 1c, a suitably occlusive device can be fabricated by simply using shorter wire-like members 12, extending from radiopaque marker band 14. This alternate embodiment (not shown) would achieve occlusion with a shorter, more compact device. The end of this device carrying the radiopaque marker band, would be placed upstream of the fluid flow with respect to the opposite end of the frame carrying membrane 18 and/or fibers 16.

With reference to Figures 1a and 1b, the manner in which the occlusion device is deployed will now be described. Wire-like members 12 of frame 10 are initially formed in the configuration depicted in Figure 1b. The ends of wires 12 are then resiliently compressed or straightened and placed within catheter 24. Piston 28 and piston rod 26 is advanced until it abuts one end of the occlusion device. A guidewire is then advanced to the desired deployment site within the vasculature. The entire system is then loaded onto the guidewire and advanced over the guidewire to the deployment site in vessel 20. The occlusion device is then held in place with piston 28 while the catheter is withdrawn. Alternatively, catheter 24 is held stationary while piston 28 is advanced. This releases the occlusion device, allowing the ends of the wires to expand into engagement with the inner wall 22 and anchor the device within vessel 20.

If, however, the wires 12 are fabricated from a shape memory alloy, such as Nitinol, fabrication and loading of the occlusion device are somewhat different. But performed in a manner well known in the art. The Nitinol wires are first heated to their transition temperature. The wires are then manipulated into the configuration shown in Figure 1b --where the ends of the wires bow outwardly. Next, the wires are heated until their crystal structure assumes its high-temperature austenite phase. The wires are then cooled until the atoms rearrange themselves into the martensite phase. The wires may now be straightened, and the occlusion device loaded into the catheter as shown in Figure 1a. Because the wires are formed from Nitinol, they will revert to their original or bowed out configuration when heated to the temperature at which they transition to the austenite phase.

Herein, the transition temperature of the Nitinol wires is preferably above room temperature and at or near body temperature. In this manner, when the system is advanced to the deployment site and the catheter is withdrawn, the frame will revert to its original configuration and engage and anchor the occlusion device to the inner wall of the vessel. Those skilled in the art will appreciate that the transition temperature of Nitinol can be manipulated over a wide range by altering the nickel-titanium ratio, by adding small amounts of other elements, and by varying the deformation and annealing processes. similarly, those skilled in this art would readily recognize that the superelastic properties of Nitinol may also be employed.

Turning now to Figures 2a-c, a second version of the present invention will now be discussed. With specific reference to Figure 2a, the occlusion device is shown in its initial configuration wherein wire-like members 12', of frame 10', are crimped together at both ends with radiopaque marker bands 14. Wires 12' have a thickness which preferably range from 1 to 20 mils. Although, thicknesses less than 1 mil and greater than 20 mils are contemplated by the present invention.

Radiopaque marker bands 14 can also be welded or soldered onto the ends of the wires 12'. Alternatively, both ends of wires 12' may be welded or soldered together and at least a portion of the surface of frame 10' can be coated or plated with a radiopaque material.

Filamentary strands, fibers or fiber bundles 16 are intertwined about and around wires 12'. A substantially non-compliant membrane similar to that shown at 18 in Figure 1c, may also be applied to wires 12'.

Wires 12' are bent or bowed outwardly intermediate the ends thereof. Frame 10' is resiliently compressed, loaded into the catheter, and deployed at the desired location in the vessel in a manner similar to that discussed concerning the embodiment of Figures 1a-c above.

Alternatively, if the wires are fabricated from a shape memory alloy such as Nitinol, the occlusion device is also formed and deployed in a manner as discussed previously with respect to Figures 1a-c. Specifically, wires 12' are heated to their transition temperature, manipulated into the bowed out configuration shown in Figure 2a, heated to the austenite phase, cooled to the martensite phase, and then flattened or straightened and loaded into the catheter 24.

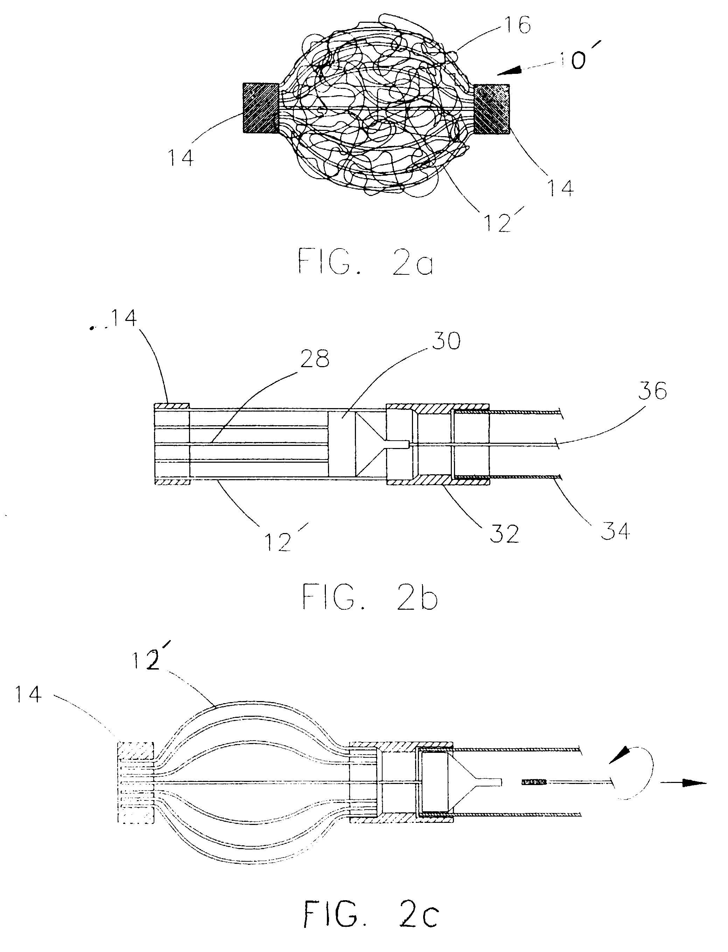

Figures 2b and 2c show a locking version of the occlusion device depicted in Figure 2a. Wire-like members 12' are crimped together at their distal end, preferably by a radiopaque marker band 14. A central wire-like member 28 includes a sliding lock 30 at the proximal end thereof. The proximal ends of wires 12' abut an internal flange of a stationary lock 32. (Stationary lock 32 is preferably made from a radiopaque material similar to marker band 14.) In turn, the opposite side of the internal flange of stationary lock 32 abuts a flexible hypotube 34. Sliding lock 30 threadingly engages a detachable locking mandrel 36.

The locking version of the occlusion device is deployed by first advancing it to the predetermined site within vessel 20. The system is then held in place while the locking mandrel is retracted until sliding lock 30 seats against the proximal side of the internal flange of stationary lock 32, as shown in Figure 2c. Alternatively, locking mandrel 36 can be held in place while hypotube 34 is advanced until sliding lock 30 seats against stationary lock 32. Locking mandrel 36 is then detached from sliding lock 30 and removed along with hypotube 34. As with the embodiment discussed above with reference to Figure 2a, filamentary strands, fibers or fiber bundles are intertwined around and about wires 12'.

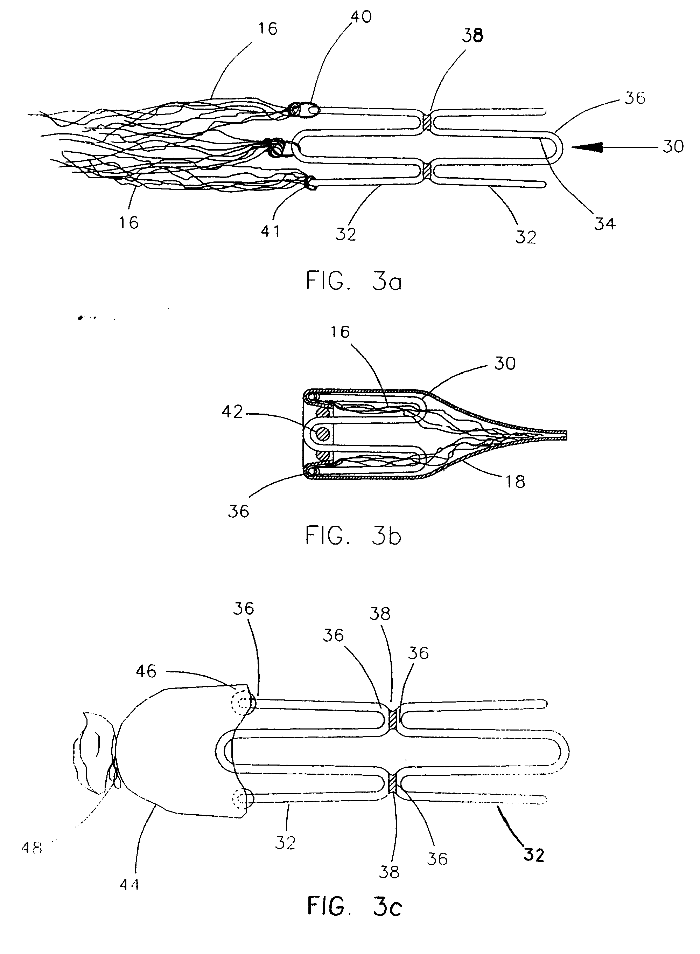

In Figures 3a-f, the anchor comprises a generally cylindrical, tubular shaped frame 30. Tubular frame 30 can include one or more segments 32. Each segment 32 comprises a single wire bent into a plurality of substantially straight sections or struts 34. These struts 34 are integrally joined by discrete axial turns or crowns 36 to form a generally cylindrical, tubular segment 32, having a sinusoidal-shaped configuration.

If two or more segments are used, the segments 32 are then joined at one or more aligned crowns by welds 38 or other known means of connection such as, by encapsulation in a polymer to allow for maximum axial and radial flexibility. To cause occlusion of flow through vessel 20, one or a plurality of filamentary strands 16 are affixed to crowns 36.

In the embodiment of Figure 3a, two fiber bundles 16 are secured to the crowns 36 in different fashion. One fiber bundle is secured to a crown 36 by fastening thread 40. The second fiber bundle is looped around a crown 36 and secured to the tubular segment 32 by using the filamentary strands themselves to form a knot 41. Longer lengths of the filamentary strands can be used to increase the thrombogenic-inducing area of the occlusion device.

It should be understood that the filamentary strands or fiber bundles can be secured to the stent in any suitable matter, and that the manner in which the filamentary strands are secured to the anchor depicted in Figure 3a is only exemplary. Any suitable means of securing the strands or fiber bundles to the tubular anchor can be used, including adhesives, heat bonding, chemical bonding, mechanical fastening or combinations thereof. Similarly, the strands or fiber bundles can be secured at other portions of the tubular anchor than the crowns 36. For example, one strand or strands can be tied to the welds 38 with another bundle secured to crowns 36.

The fastening thread 40 can comprise the same material as the filamentary strands or a different suitable material. Thus, the thread fasteners 40 can advantageously comprise thrombogenic elements or a material which does not promote thrombogenic occlusion. The filamentary strands or fiber bundles 16 employed in this embodiment are made from the same group of materials discussed with reference to the embodiments of Figures 1a-c and 2a-c above.

Figure 3b shows a non-compliant or inelastic membrane 18, as discussed previously with reference to Figure lc, attached to tubular frame 30 by thermal bonding, heat shrinking, coating, adhesive, welding, induction heating, or the like. In this embodiment, however, the process of adhering membrane 18 to frame 30 includes a folding of the open end of the membrane over crowns 36. Moreover, filamentary strands or fiber bundles 16 can advantageously be secured to the anchor by capture of one end thereof within the fold of membrane 18. Additionally, a radiopaque marker or markers 42 can also be placed within the fold of membrane 18.

Figure 3c shows a further embodiment of the present invention. A substantially non-compliant element 44 is secured to the crowns 36 of the frame 30. The element 19 is a fabric cylinder, made of a thrombogenic inducing material. One end is tied to the crowns by thread fasteners 46 with the other end secured by thread fastener 48. Although the thrombogenic element 44 is shown threadably attached to the left end crowns 36 in Figure 3c, it can be sized to enclose substantially the entire outer surfaces of frame 30 and threaded to the free ended crowns opposite those providing attachment in Figure 3c.

Specific embodiments of the present invention depicted in Figures 3a and 3b comprise a generally cylindrical, tubular anchor having an expanded or deployed diameter of about 3.5 mm and a length of about 18 mm with a 0.002 inch polyethylene terephthalate monofilament as the thrombogenic inducing element. The polyethylene terephthalate monofilament can be hand wound around the outer surfaces of the entire length of the anchor and tied at one of the free end crowns. The anchor is then ready for positioning into a vessel at a predetermined location. Alternatively, anchor 30 can be coated with polyethylene terephthalate, as the substantially non-compliant membrane, with or without polyethylene terephthalate monofilament strands secured to the crowns or, if the polyethylene terephthalate is folded over the crowns, secured to the anchor by the membrane itself.

In a further example of the present invention, a polyethylene terephthalate spun yarn in the as received condition was split from one strand into three. Each split strand was further split into seven strands for a total of 21 strands. Each of the twenty-one strands was then split into four strands for a total of 84 strands. Each of the 84 strands was then threaded around the stent crown and looped back on itself. A polyethylene thread was then used to tie the yarn so that it remains on the stent crown. Three surgeon's knots were used to attach each of the split yarns to a crown. This procedure is repeated four times to produce four thrombogenic elements extending from one end of the stent. In this example, yarn splitting enables a lower delivery profile combined with a greater amount of material, i.e. surface area, for effecting thrombogenic occlusion.

In yet another example, the yarn is split along only a portion of the yarn length, thereby dividing the filamentary strand into a non-split core portion and a split portion. The core portion is then attached to the crown or other site, leaving the split portion trailing the anchor for thrombogenic occlusion. As one having ordinary skill in this art would recognize, other methods of tying and/or splitting a yarn or other fiber can be utilized to maximize thrombogenic occlusion.

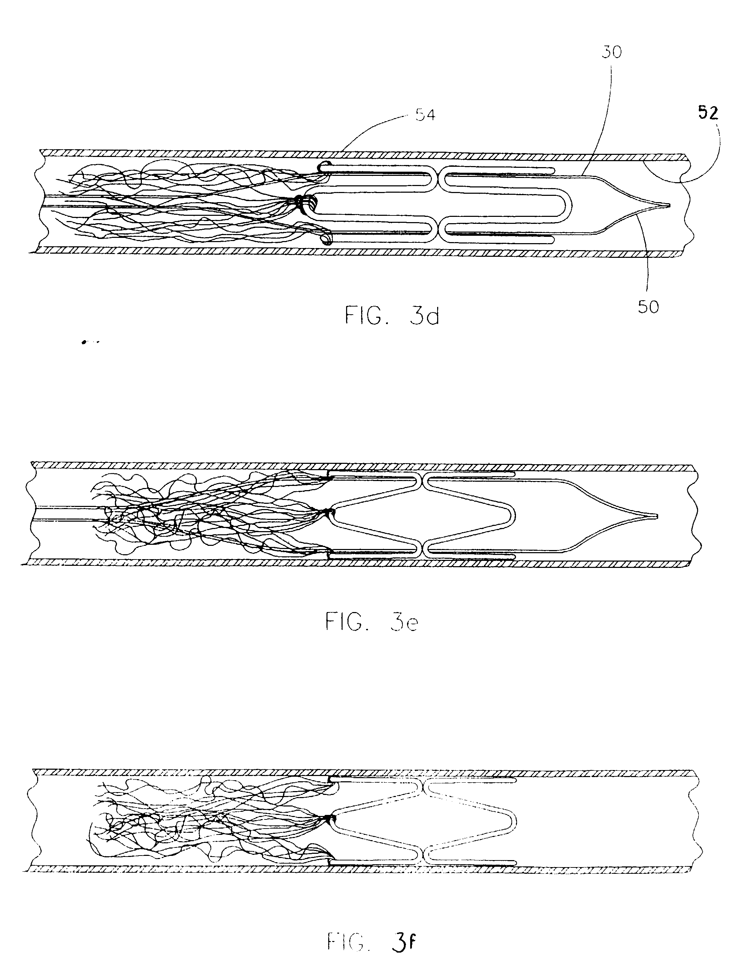

A method of disposing the occlusion devices described in Figures 3a-c, in a vessel is schematically depicted in Figs. 3d-f. Referring to Fig. 3d, the occlusion device 30 is disposed on a balloon catheter 50 and advanced to a predetermined location 52 for intravascular occlusion of vessel 54. Conventional imaging techniques and radiopaque dyes can be employed to facilitate accurate positioning of occlusion device 30 at site 52.

As shown in Fig. 3e, upon positioning the catheter at site 52, the balloon is inflated to expand the occlusion device 30 into engagement with the inner annular wall of vessel 54. The anchor is expanded past its proportional plastic limit so that it remains in the expanded configuration and in contact with vessel 54, to secure itself in place at site 52. The balloon is then deflated and removed, as shown in Fig. 3f. In such a manner, the anchor is embedded in the inner wall of the vessel 54, remaining secured in place, permitting the occluding element, or elements, attached thereto to cause occlusion.

The use of balloon catheters to dispose anchors 30 in vessels is similar to that which is commonly used to deploy stents, and therefore, is well known in the art. Consequently, a detailed description thereof is not considered necessary for an understanding of the present invention. As one having ordinary skill in the art would recognize, other delivery systems can be used to dispose the inventive occlusion device at a predetermined site in a vessel.

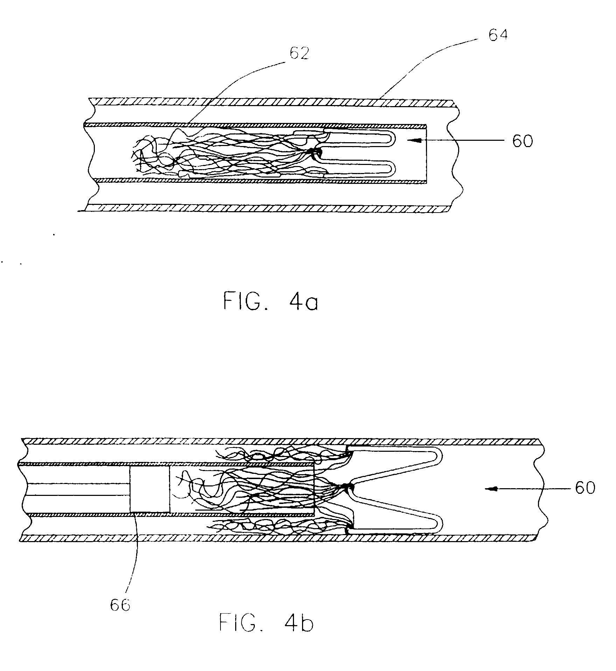

Referring to Figures 4a and 4b, disposition of a self-expanding intravascular occlusion device is depicted. The self-expanding intravascular occlusion device is designated by the reference numeral 60 and is shown disposed within a catheter 62. One or more filamentary strands or fiber bundles are secured to the crowns of device. The catheter is then advanced to a predetermined site within a vessel 64. Once the catheter is positioned at the predetermined location for intravascular occlusion, a pusher 66 is inserted within the catheter 62 and the occlusion device 60 is pushed out from within the confines of the catheter 62. Without restraint by the catheter, the occlusion device 60 undergoes self-expansion and is seated securely on the interior wall of the vessel 64 as shown in Figure 4b. The catheter 62 and pusher 66 are then withdrawn, leaving the accurately positioned occlusion device secured in place for its intended function. The self-expanding feature of the occlusion device can be achieved employing a generally cylindrical, tubular-shaped anchor comprising a spring steel which is compressed by the catheter 62 and then expands when expelled therefrom. Alternatively, the occlusion device 60 can comprise a shape memory alloy, e.g., a nickel-titanium alloy, which will resume its memory shape in Figure 4b upon expulsion from the catheter 62, as discussed above with reference to Figures 1a-c. As one having ordinary skill would recognize, any suitable self-expanding material can be employed for the anchor portion of the inventive occlusion device.

The intravascular occlusion device of the present invention can be employed to effect occlusion in any vessel. As one having ordinary skill would recognize, the anchor profile or diameter, length and material, can be varied depending on the particular application, with the consideration to the size of the vessel. The type of occlusive element, manner of element attachment and coating can also be varied to suit a particular application. For example, in very small vessels, only one occlusive element may be required to cause occlusion and thrombosis; whereas, larger vessels may require more elements to effect occlusion.

In accordance with the present invention, the occlusion time can be optimized for a particular size vessel by varying the diameter and length of the anchor. A high loading of the occlusive element, or elements, can be used for rapid occlusion. In accordance with the present invention, in cranial vessels having a diameter ranging from approximately 1.0 mm to 5.0 mm, total occlusion can be effected in approximately 0.25 to 20 minutes, preferably, in less than one to three minutes.

As such, an invention has been disclosed in terms of preferred embodiments thereof which fulfill each and every one of the objects of the present invention as set forth above and provides a new and improved intravascular occlusion device and method of use.

Various changes, modifications and alterations from the teachings of the present invention may be contemplated by those skilled in the art without departing from the intended spirit and scope thereof.

More specifically, a further aspect of the instant invention comprises coating or impregnating fiber bundles 16 and/or membrane 18 with a material which enhances their thrombogenic efficacy. Examples of which include collagens, peptides, polymers, thrombins and proteins.

Moreover, the fiber bundles and/or membrane may be coated or loaded with a pharmaceutical agent enabling local drug delivery. For example, a chemotherapeutic agent can be included in a polymer, as by encapsulation or impregnation. The polymer-coated element, is then placed in the primary feeding vasculature of a tumor. This technique advantageously enables accurate, local delivery of toxic drugs and effective disease treatment without the morbidity associated with systemically administered drug levels. Simultaneously, the occlusive properties of the device eliminates nourishment to the tumor and minimizes wash out of the drug.

In addition, an aspect of the present invention includes treating the surface of frames 10, 10', 30 or 60 so as to induce a thrombogenic effect. This treatment may take the form of coating the surface of the frame, or a portion thereof, with a thrombogenic material or roughening the surface, or at least those portions of the frame surface in contact with the vessel, such as by mechanical roughening, chemical etching or laser process.

Also, the disclosed manner of folding the occlusive membrane over the end of the crowns, as described with respect to Figure 3b, is equally applicable to the wire-like frames disclosed in Figures 1a-c. Likewise, an occlusive membrane, either in combination with or instead of the fibers, may be applied in a similar manner to the self-expanding tubular frame depicted in Figures 4a and 4b.

## Claims

1. A device for occluding fluid flow through a vessel, comprising:
an anchor; and
a substantially non-complaint membrane affixed to said anchor.

2. A device as claimed in claim 1, wherein the membrane is coated or loaded with a pharmaceutical agent.

3. A device as claimed in claim 1, wherein the membrane comprises a material selected form the group consisting essentially of polyethylene, polyethylene terephthalate, polyarylenesulfide and polyamides.

4. A device as claimed in claim 1, further comprising at least one filamentary strand affixed to said anchor.

5. A device as claimed in claim 4, wherein the at least one filamentary strand comprises a material selected from the group consisting essentially of silk, cotton, wool, cellulose, nylon, polyethylene, polyethylene terephthalate, polypropylene, polyester, polyurethane, collagen and metals.

6. A device as claimed in claim 4, wherein the at least one filamentary strand is coated with a material which enhances thrombosis.

7. A device as claimed in claim 4, wherein the at lest one filamentary strand is impregnated with a material which enhances thrombosis.

8. A device as claimed in claim 6 or claim 7, wherein the thrombosis enhancing material is selected from the group consisting essentially of collagens, peptides, polymers, thrombins and proteins.

9. A device as claimed in claim 4, wherein the at least one filamentary strand is coated or loaded with a pharmaceutical agent.

10. A device as claimed in claim 1, wherein the anchor comprises a material selected from the group consisting essentially of stainless steel, nickel, titanium, nickel-titanium, Nitinol, tantalum, cobalt-chromium, copper and gold.

11. A device as claimed in claim 1, wherein the anchor is treated to induce thrombosis.

12. A device as claimed in claim 1, wherein the anchor includes a frame comprising a plurality of wire-like members, each member having a first and second end. And wherein the plurality of wire-like members are crimped together with a radiopaque marker band intermediate of the first and second ends of said members.

13. A device as claimed in claim 1, wherein the anchor further comprises a plurality of wire-like members, each member having a first and second end, the wire-like members further comprising:
a first configuration and a second, deployed configuration;
a first radiopaque marker band securing the first ends of the wire-like members together;
a second radiopaque marker band securing the seconds ends of the wire-like members together; and
means for locking said plurality of wire-like members in the second, deployed configuration.

14. A device as claimed in claim 1, wherein the anchor further comprises:
plurality of substantially straight segments connected at axial bends; and
at least one filamentary strand connected to at least one of said axial bends.

15. A device as claimed in claim 1, wherein the anchor further comprises;
a tubular, generally cylindrical member having first and second ends, and
a portion of the membrane is folded over one end of the tubular member.

16. A device as claimed in claim 15, further comprising:
at least one filamentary strand affixed to the device by capture of at least one end thereof within said folded portion of the membrane.

17. A device as claimed in claim 15, further comprising:
at least one radiopaque marker placed within the folded portion of the membrane.

18. A device as claimed in claim 1, wherein the anchor further comprises:
a plurality of substantially straight segments connected at axial bends; and
at least one filamentary strand connected to at least one of said axial bends.

19. A device as claimed in claim 1, wherein the anchor comprises:
a generally cylindrical, tubular member made of a shape memory material and having a first diameter and a transition temperature at or substantially near body temperature:
said tubular member having a second, deformed diameter which permits intraluminal delivery of the tubular member to the vessel to be occluded; and
means for restraining said tubular member in the second, deformed diameter until the device is positioned at a predetermined location in the vessel, whereby upon removal of the restraining means the tubular member transition to the first diameter, engaging the inner wall of the vessel and maintaining said device at said predetermined location in said vessel.

20. A device for occluding fluid flow through a vessel, comprising:
an anchor; and
at least one filamentary strand affixed to said anchor.

21. A device as claimed in claim 20, wherein the at least one filamentary strand comprises a material selected form the group consisting essentially of silk, cotton, wool, cellulose, nylon, polyethylene, polyethylene terephthalate, polypropylene, polyester, polyurethane, collagen and metals.

22. A device as claimed in claim 20, wherein the at least one filamentary strand is coated with a material which enhances thrombosis.

23. A device as claimed in claim 20, wherein the at least one filamentary strand is impregnated with a material which enhances thrombosis.

24. A device as claimed in claim 22 or claim 23, wherein the thrombosis enhancing material is selected from he group consisting essentially of collagens, peptides, polymers, proteins.

25. A device as claimed in claim 20, wherein the at least one filamentary strand is coated or loaded with a pharmaceutical agent.

26. A device as claimed in claim 20, wherein the anchor is treated to induce thrombosis.

27. A device as claimed in claim 20, wherein the anchor includes a frame comprising a plurality of wire-like members, each member having a first and second end.

28. A device as claimed in claim 27, wherein the plurality of wire-like members are crimped together with a radiopaque marker band intermediate of the first and second ends of said members.

29. A device as claimed in claim 27, wherein the frame comprises a material selected from the group consisting essentially of stainless steel, nickel, titanium, nickel-titanium, Nitinol, tantalum, cobalt-chromium, copper and gold.

30. A device as claimed in claim 27, wherein the wire-like members have a first configuration and a second, deployed configuration whereby, in said second configuration, those portions of the wire-like members intermediate the first and second ends thereof, engage the inner wall of the vessel and maintain said anchor at a predetermined location within said vessel.

31. A device as claimed in claim 30, further comprising:
a first radiopaque marker band securing the first ends of the wire-like members together;
a second rediopaque marker band securing the second ends of the wire-like members together; and
means for locking said plurality of wire-like members in the second, deployed configuration.

32. A device as claimed in claim 20, wherein the anchor further comprises:
a plurality of substantially straight segments connected at axial bends; and
said at least one filamentary strand being connected to said tubular member at one of said axial bends.

33. A device as claimed in claim 20, wherein the anchor comprises a material selected form the group consisting essentially of stainless steel, nickel, titanium, tantalum, cobalt-chromium, copper and gold.

34. A device as claimed in claim 20, wherein the anchor comprises:
a generally cylindrical, tubular member made of a shape memory material and having a first diameter and a transition temperature at or substantially near body temperature;
said tubular member having a second, deformed diameter which permits intraluminal delivery of the tubular member to the vessel to be occluded; and
means for restraining said tubular member in the second, deformed diameter until the device is positioned at a predetermined location in the vessel, whereby upon removal of the restraining means the tubular member transitions to the first diameter, engaging the inner wall of the vessel and maintaining said device at said predetermined location in said vessel.

35. A device as claimed in claim 34, wherein the tubular member further comprises:
a plurality of substantially straight segments connected at axial bends; and
said at least one filamentary strand is connected to said tubular member at one of said axial bends.

36. A device as claimed in claim 34, wherein the shape memory material is nickel-titanium or an alloy thereof.

37. An intravascular occlusion device comprising;
means for thrombogenically occluding flow through a vessel; and
means for maintaining said flow occluding means at a predetermined location within said vessel.

38. The intravascular occlusion device according to claim 37, further comprising:
means for inducing a thrombogenic response form said maintaining means.

39. The intravascular occlusion device according to claim 37, further comprising:
means for increasing the thrombogenicity of said flow occluding means.

40. The intravascular occlusion device according to claim 37, further comprising:
means for securing said flow occluding means to said maintaining means.

41. The intra vascular occlusion device according to claim 37, further comprising:
means for delivering the maintaining means and the flow occluding means to a predetermined location in a vessel to be occluded.

42. The intra vascular occlusion device according to claim 37, further comprising:
means for delivering a pharmaceutical agent to the vessel.

43. An intra vascular occlusion device comprising:
a substantially non-compliant means for mechanically occluding flow through a vessel; and
means for maintaining said mechanical flow occluding means at a predetermined location within said vessel.
